# EUROPEAN PATENT SPECIFICATION

(11) **EP 0 554 352 B1**
(45) Date of publication and mention of the grant of the patent: **05.03.2003**
(21) Application number: 91919823.4
(22) Date of filing: 09.10.1991
(51) Int. Cl.: C12N 5/06, C12N 5/08, C12N 5/10, A61K 35/12

(54) **A METHOD FOR TRANSPLANTING CELLS INTO THE BRAIN AND THERAPEUTIC USES THEREFOR**
EINE METHODE ZUR TRANSPLANTATION VON ZELLEN IN DAS GEHIRN UND IHRE THERAPEUTISCHE VERWENDUNG
PROCEDE DE TRANSPLANTATION DE CELLULES DANS LE CERVEAU ET UTILISATIONS THERAPEUTIQUES DU PROCEDE

(30) Priority: 19.10.1990 US 599802
(43) Date of publication of application: 11.08.1993
(73) Proprietor: NEW YORK UNIVERSITY, New York, NY 10016 (US)
(72) Inventor: CHERKSEY, Bruce, D., Hoboken, NJ 07030 (US)
(74) Representative: Brasnett, Adrian Hugh
(86) International application number: US9107443
(87) International publication number: WO92006702

(56) References cited:
- WO-A-90/02796
- WO-A-90/12604
- US-A- 4 748 121
- US-A- 4 892 538
- US-A- 4 900 553
- FILE SERVER STN KARLSRUHE,FILE MEDLINE ABSTRACT NO.88055485 & EXP NEUROL, (1987 DEC) 98(3) 606-15
- FILE SERVER STN KARLSRUHE,FILE MEDLINE ABSTRACT NO.87206236 & SCIENCE, (1987 MAY 29) 236(4805) 1106-9
- Methods in Enzymology, Volume LVIII, issued 1979, THILLY et al., "Microcarrier Culture: A Homogeneous Environment for Studies of Cellular Biochemistry", pages 184-186, see the entire document.
- Science, Volume 242, issued 16 December 1988, ROSENBERG et al., "Grafting Genetically Modified Cells to the Damaged Brain: Restorative Effects of NGF Expression", pages 1575-1577, see entire document.

## Description

### BACKGROUND OF THE INVENTION

### Field of the Invention

The invention in the field of neuroscience and medicine relates to methods for implantation or transplantation of cells into the mammalian brain, useful in treating neurological disorders.

### Description of the Background Art

Grafting of neural cells and neuron-like cells directly into the brain may be a practical approach to promote recovery from debilitating diseases of the nervous system such as Parkinson's disease (PD), Huntington's disease (HD), Alzheimer's disease (AD), epilepsy, and familial dysautonomia, as well as injury or trauma to the nervous system. The characterization of factors which influence neurotransmitter phenotypic expression in cells placed into the brain may lead to a better understanding of processes involved in normal specification in the nervous system and indicate means by which birth defects resulting from aberrant phenotypic expression can be therapeutically prevented or corrected.

Neurons or neuronal-like cells can be grafted into the central nervous system (CNS), in particular, into the brain, either as solid tissue blocks or as dispersed cells. However, to date, a number of problems of both a technical and ethical nature have plagued the development of clinically feasible grafting procedures.

Parkinson's disease (PD) results from a selective loss of dopaminergic nigrostriatal neurons, resulting in a loss of input from the substantia nigra to the striatum.Solid grafts of tissues potentially capable of producing dopamine, such as adult adrenal medulla and embryonic substantia nigra (SN), have been used extensively for experimental grafting in rats and primates treated with 6-hydroxydopamine (6-OHDA) to destroy dopaminergic cells (Dunnett, S.B. et al., Brain Res. 215: 147-161 (1981); ibid. 229:457-470 (1981); Morisha, J.M. et al., Exp. Neurol. 84:643-654 (1984); Perlow, M.J. et al., Science 204:643-647 (1979)). Grafts of embryonic SN have also been used as therapy for primates lesioned with the neurotoxin 1-methyl-4-phenyl-1,2,3,4-tetrahydropyridine (MPTP), which produces a PD-like disease (Redmond, D.E. et al., Lancet 8490:1125-27 (1986)).

Stenevi et al. (Brain Res. 114:1-20 (1976), in investigating the optimal conditions for neural graft survival, found that the best results were obtained when (a) fetal CNS neurons were used, and (b) the transplanted cells were placed next to a rich vascular supply. In fact, a review of the literature reveals that tissue from almost every area of the fetal brain can be successfully transplanted if care is taken with procedural details (see, for example, Olson, L. A. et al., In: Neural Transplants: Development and Function, Sladek, J.R. et al., eds, Plenum Press, New York, 1984, pp. 125-165).

Embryonic tissue provides an excellent source of cells which will differentiate in a foreign environment and become integrated with the host tissue. For example, grafts of embryonic SN into 6-OHDA treated rats have been shown to produce dopamine, to reduce apomorphine- or amphetamine-induced rotation, to alleviate sensory deficits and to make synapses in the host striatum (Dunnett et al., Morisha et al., Perlow et al., supra). Grafted neurons are also spontaneously active, thus mimicking normal adult SN neurons (Wuerthele, S.M. et al., In: Catecholamines, Part B, (E. Usdin et al., eds.), A.R. Liss, Inc., New York, pp. 333-341).

In contrast to successful grafting of fetal neural tissue, mature CNS neurons have never been found to survive in transplants (Stenevi, U. et al., Brain Res. 114:1-20 (1976)). The reason fetal CNS neurons survive grafting procedures while adult neurons do not, while uncertain, is probably related to several factors. First, fetal neurons are less affected by low oxygen levels than mature neurons (Jilek, L., In: Developmental Neurobiology, Himwich, W.A., ed., C.C. Thomas Publisher, Springfield, IL, 1970, pp. 331-369), and grafting procedures necessarily involve periods of anoxia until an adequate blood supply to the transplant is 5 established. Secondly, fetal neurons seem to survive best when they are taken during a rapid growth phase and before connections are established with target tissues (Boer, G.J. et al., Neuroscience 15:1087-1109, (1985)). Also, fetal tissue may be especially responsive to growth (or "survival") factors which are known to be present in the milieu of the damaged host brain (Nieto-Sampedro, M. et al., Science 217:860-861 (1982); Proc. Natl. Acad. Sci. USA 81:6250-6254 (1984)).

However, despite the promise of fetal tissue and cell transplants, the art has turned to alternate sources of donor tissues for transplantation because of the ethical, moral, and legal problems attendant to utilizing fetal tissue in human medicine. These sources include neural and paraneural cells from organ donors and cultured cell lines. (See, for example: Gash, D.M. et al., In: Neural Grafting in the Mammalian CNS, Bjorklund, A. et al., eds, Elsevier, Amsterdam, 1985, pp. 595-603; Gash, D.M. et al., Science 233:1420-22 (1986)).

Although early clinical experiments using the grafting approach did not result in long-lasting effects, an initial report of one study appeared more promising (Madrazo et al., Soc. Neurosci. Abstr. 12:563 (1986); for an overview, see: Lieberman, A. et al., Adv. Tech. Stand. Neurosurg. 17:65-76 (1990), which is hereby incorporated by reference). Several additional observations suggest that grafting adrenal cells should be a viable approach. Adrenal medullary cells are derived from the neural crest and, like sympathetic neurons, grow processes in vivo or in vitro in response to nerve growth factor (NGF) (Unsicker, K. et al., Proc. Natl. Acad. Sci. USA 75:3498-3502 (1978)). Solid grafts of adrenal medulla from young rats can survive in the brain of 6-OHDA treated rats for at least 5 months, produce dopamine and reduce apomorphine induced rotation (Dunnett et al., supra; Freed, W.J. et al., Ann. Neurol. 8:510-519 (1980); Freed, W.J. et al. Science 222:937-939 (1983)). These observations suggest that given the appropriate environment, adrenal medullary cells have the potential for growing catecholamine-synthesizing fibers into brain tissue.

The potential for neuronal differentiation of chromaffin cells is even better elucidated by grafts of dissociated adrenal chromaffin cells which grow processes when injected into rat striatum. Cultured adrenal medullary cells also differentiate into neuronal-like cells with processes when cultured in the presence of NGF (Unsicker, supra). This remarkable plasticity of adrenal cells is observed not only in morphology, but also in neurotransmitter phenotypic expression. An array of neuropeptides including vasoactive intestinal peptide, as well as the monoamine, serotonin, are co-localized with catecholamines in adrenal medullary cells (Schultzberg, M. Neurosci. 3:1169-1186 (1978); Lundberg, J.M. Proc. Natl. Acad. Sci. USA 76:4079-4082 (1979)). Expression of these various phenotypes can be modulated by extrinsic signals. For example, enkephalin and VIP expression are increased following denervation of the adrenal in vivo, by treatment of animals with nicotinic blockers or after maintaining adrenal cells in vitro (Tischler, A.S. Life Sci. 37:1881-1886 (1985)). These observations taken together with those of other studies demonstrating that neurons derived from the neural crest can switch phenotype during normal development (Bohn, M.C. et al., Devel. Biol. 82:1-10 (1981); Jonakait, G.M. et al. Devel. Biol. 88:288-296 (1981)) or following experimental manipulation of the micromilieu (LeDouarin, N.M. Science 231: 1515-1522 (1986)) suggest that, in the future, it may be possible to control the neurotransmitter phenotype expressed by grafted cells either before and/or after grafting.

An additional advantage of grafting dissociated cells compared to blocks of tissue is that the cells can be precultured with various substances such as growth factors prior to grafting or they can be co-grafted with other cells or substances which promote specific parameters of differentiation. Furthermore, glial cells may have specific regional effects and produce neuronal growth factors (Barbin, G. et al., Devel. Neurosci. 7:296-307 (1985); Schurch-Rathgeb, Y. et al., Nature 273:308-309 (1978); Unsicker, K. et al. Proc. Natl. Acad. Sci. USA 81:2242-2246 (1984); Whitaker-Azmitia, P.M. et al., Brain Res. 497:80-85 (1989)). This suggests that co-transplanting cells providing the desired neurotransmitters along with specific types of glia which produce glial-derived factors, may promote neuronal growth and the desired differentiation of grafted cells.

Thus, the feasibility of the transplant approach has been established experimentally. However, this approach is severely limited by the need for the use of fetal tissue, which is of limited availability and of great political consequence. In essence, transplantation of human fetal tissue from aborted pregnancies has been prohibited in the United States. It would thus be of great benefit if simple, routine and safe methods for the successful transplantation of adult tissue into the brain were available for the treatment of debilitating neurological disease.

Aebischer and his colleagues have successfully implanted selectively permeable biocompatible polymer capsules into the brain which encapsulated fragments of neural tissue which survived in this environment (Aebischer, P. et al., Brain Res. 448:364-368 (1988); Winn, S.R. et al., J. Biomed Mater Res. 23:31-44 (1989). The polymer capsules consisted of polyvinyl chloride acrylic copolymer XM-50. Such permselective material completely prevented the invasion of the encapsulated tissue by host cells, and presumably antibodies and viruses as well, based on the permeability of the membrane. When dopamine-releasing polymer rods were encapsulated into such a permselective polymer and implanted into denervated striatum in rats, alleviation of the experimentally induced Parkinson disease symptoms was achieved (Winn S.R. et al., Exp. Neurol. 105:244-50 (1989). Furthermore, US Patent 4,892,538 (Aebischer et al., issued 1/9/90) discloses a cell culture device for implantation in a subject for delivery of a neurotransmitter comprising secreting cells within a semipermeable membrane which permits diffusion of the neurotransmitter while excluding viruses, antibodies and other detrimental agents present in the external environment. The semipermeable membrane is of an acrylic copolymer, polyvinylidene fluoride, polyurethane, polyalginate, cellulose acetal, polysulphone, polyvinyl alcohol, polyacrylonitrile, or their derivatives or mixtures and permits diffusion of solute of up to 50 kD molecular weight. This device is said to be useful in treatment of neurotransmitter-deficient conditions, such as Parkinson's disease, by sustained, local delivery of neurotransmitters, precursors, agonists, fragments, etc., to a target area, especially the brain. The device may be made retrievable so that the contents may be renewed or supplemented, and the cells are protected against immunological response and viral infection.

### SUMMARY OF THE INVENTION

The inventors have made the unexpected discovery that by first culturing cells in vitro on a support matrix, such as 90 µm diameter glass beads, such cells including mature CNS neurons or cultured cells, can be successfully transplanted into the mammalian brain. The beads, to which the cells adhere, are stereotaxically injected into the recipient's brain. Cells so injected retain their viability and are thus effectively transplanted. They show prolonged survival and viability in vivo, even when transplanted across species barriers.

It is an objective of the present invention to overcome the aforementioned deficiencies in the prior work.

The present invention provides a method for grafting a cell in the brain of a mammalian subject comprising culturing the cell with a support matrix so that the cell attaches to said matrix, and implanting the support matrix with the attached cell into the brain.

The method includes support matrices made of glass and other silicon oxides, polystyrene, polypropylene, polyethylene, polyacrylamide, polycarbonate, polypentene, acrylonitrile polymers, nylon, amylases, gelatin, collagen, natural and modified polysaccharides, including dextrans and celluloses (e.g. nitrocellulose), agar, and magnetite. 5 Preferred support matrices are beads, in particular microbeads having a diameter from about 90 to about 150 µm.

The method of the present invention may employ cells of many different types, preferably either cells of neural or paraneural origin, such as adrenal chromaffin cells. Also useful are cell lines grown in vitro. Cells not of neural or paraneural origin, such as fibroblasts, may also be used following transfection with DNA encoding a neuropeptide or an enzyme or set of enzymes which results in production of neurotransmitter, or a neuronal growth factor.

The present invention includes a method for treating a neurological disease in a subject which comprises grafting an effective number of cells capable of treating the disease according to the above methods. Diseases which can be treated according to the present invention include, but are not limited to, Parkinson's disease, Alzheimer's disease, Huntington's disease, epilepsy, familial dysautonomia, and traumatic brain injury.

### BRIEF DESCRIPTION OF THE DRAWINGS

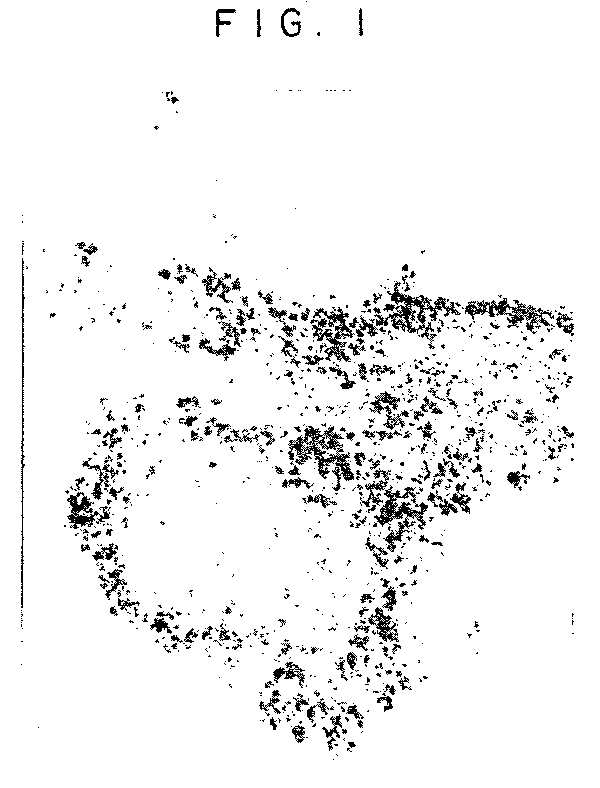

Figure 1 is a photomicrograph of a section of the brain of a rat implanted with adult adrenomedullary cells on a glass microbead. The section was fixed an stained histochemically for tyrosine hydroxylase (TH) using horse radish peroxidase. The darkly stained areas indicate cells containing TH. The pattern of staining shows a needle tract entering the section on the right side. The large circular stained area in the center represents the bulk of the implanted cells. Te injected cells form a "gland-like" pattern, with some extending back into the needle tract. (Thickness: 20 microns; magnification: 100x)

### DESCRIPTION OF THE PREFERRED EMBODIMENTS

The present invention is based on the unexpected discovery by the inventors that adult cells or cultured cells which cannot normally be transplanted into a mammalian brain and survive, can be made to survive by first attaching them to a support matrix.

A number of different cell types are useful for the present invention. Typically, a cell will be selected based on its ability to provide a missing substance to the recipient brain. Missing substances can be neurotransmitters or other neurally-active molecules, the absence of which results in neurological disease or dysfunction. It is important that the transplanted cell not grow as a tumor once it is inserted into the recipient.

The references cited below are all incorporated by reference herein, whether specifically incorporated or not.

By the term "neural or paraneural origin" is intended a cell which is derived from the embryonic neural crest. A preferred example of a cell of paraneural origin is a adrenal medullary chromaffin cell. The precursor cells to the mammalian adrenal medulla are of neural crest origin and possess the potential to develop along either neuronal or endocrine lines of differentiation (Bohn, M.C. et al., 1981, supra, Devel. Biol. 89:299-308 (1982); Unsicker, K., Develop. Biol. 108:259-268 (1985)). Chromaffin cells from the rat, monkey, and human adrenal medulla, when removed from adrenal cortical influences and exposed to nerve growth factor (NGF), change from an endocrine to a neuronal phenotype (Notter, M.F. et al., Cell Tiss. Res. 244:69-?0 (1986); Stromberg, I. et al., Exp. Brain Res. 60:335-349 (1985); Unsicker, K. et al., 1978, supra). When co-grafted with cerebral cortical or hippocampal tissue into the anterior chamber of the rat eye, adrenal chromaffin cells form nerve fibers which innervate the adjacent co-grafted brain tissue (Olson, L. A. et al., Exp. Neurol. 70414-426 (1980)). Another paraneural cell type is a retinal pigment epithelium cell (Song, M-K et al., J. Cell. Physiol. 148:196-203 (1990)).

One source of donor cells are established neural cell lines. Many neuronal clones exist which have been used extensively as model systems of development since they are electrically active with appropriate surface receptors, specific neurotransmitters, synapse forming properties and the ability to differentiate morphologically and biochemically into normal neurons. Neural lines may express a tremendous amount of genetic information which corresponds to the genetic expression seen in CNS neurons. Such cells are described in the following references: Kimhi, Y. et al., Proc. Natl. Acad. Sci. USA 73:462-466 (1976); In: Excitable Cells in Tissue Culture, Nelson, P.G. et al., eds., Plenum Press, New York, 1977, pp. 173-245); Prasad, K.M. et al., In: Control of Proliferation of Animal Cells, Clarkson, B. et al., eds., Cold Spring Harbor Laboratory Press, Cold Spring Harbor, NY, 1974, pp. 581-594); Puro, D.G. et al., Proc. Natl. Acad. Sci. USA 73:3544-3548 (1976); Notter, M.F. et al., Devel. Brain Res. 26:59-68 (1986); Schubert, D. et al., Proc. Natl. Acad. Sci. USA 67:247-254 (1970); Kaplan, B.B. et al., In: Basic and Clinical Aspects of Molecular Neurobiology, Guffrida-Stella, A.M. et al., eds., Milano Fondozione International Manarini, 1982)).

A major advantage of these cultured cells has been the potential to manipulate the environment, as well as the cells themselves, in controlling the phenotype and genotype.

For example, human neuroblastoma cells from the IMR 32 cell line can survive and express cholinergic markers in primate brain nine months after transplantation (Gash, D.M. et al., Science 233:1420-22 (1986)). These cells are preferably treated to render them morphologically and biochemically differentiated in vitro and must be rendered permanently amitotic before implantation, which further aids in their survival (Gash et al., supra; Gupta, M. et al., Dev. Brain Res. 19:21-29 (1985)).

Whereas untreated pheochromocytoma/neuroblastoma cells (PC12 cells) which grafted into the adult rat striatum showed no survival after two weeks in vivo (Hefti, F. et al., Brain Res. 348:283-288 (1985)), treatment in vitro with NGF to stimulate neural cell differentiation followed by exposure to antimitotic agents to inhibit cell proliferation, does not deleteriously affect catecholamine neurotransmitter expres-sion nor viability and is of value for stimulating performance following transplant. Therefore, in one embodiment of the present invention, cell line cells are modulated in vitro with the appropriate growth or differentiation factor and with an amitotic agent before transplantation in order to promote cell survival and prevent expression of the malignant potential.

It will be apparent to one of ordinary skill in the art that examination of the neural cell surface of clonal cells in vitro using conventional methods will permit the selection of appropriate cells for use according to the present invention. During normal development and differentiation, the neuronal cell surface undergoes significant changes which account for the migration, recognition and integration of neurons in the CNS. For example, when mouse C1300 clonal, neural cell lines are treated with antimitotic agents or growth modulators such as prostaglandin E₁ and cyclic AMP, new low molecular weight cell surface glycoproteins are produced (Bottenstein, J.E., In: Functionally Differentiated Cell Lines, Sato, G.H., ed., Alan R. Liss, New York, 1981). CNS surface gangliosides are induced which bind tetanus toxin (Notter, M.F., 1986, supra) while specific lectin binding glycoproteins appear which are similar to normal central nervous system glycoproteins. Additionally, receptors for neurotransmitters and neuropeptides on neuroblastoma cells can be modified in a manner similar to cells found in the CNS (Costa, L.G. et al., Biochem. Pharmacol. 91:3407-3413 (1982).

Another important source of potential graft material are cells engineered by somatic cell hybridization, a process which can immortalize single neurons. Somatic cell hybridization is a powerful cell biologic tool used not only to generate cell lines with a variety of genotypes but to analyze the mechanisms regulating the expression of various phenotypes of differentiation. Fusing cells which differ in the expression of specific genes allows for the exploration of the mechanisms controlling gene expression while chromosome alterations occur at rates to generate genetically different cell lines. Hybrid cells can be formed which retain the properties of differentiated cells. Hybrids derived from fusion of sympathetic ganglia and neuroblastoma cells can synthesize dopamine (Greene, L.A. et al., Proc. Natl. Acad. Sci. USA 82:4923-4927 (1975) while brain cell hybrids express choline acetyltransferase (Minna, J.D. et al., Genetics 79:373-383 (1975)). Therefore, embryonic precursors to dopaminergic neurons from the CNS can be fused with mitotic cells to incorporate both genomes into a single one that loses extra chromosomes over time and results in a new hybrid line. It is within the skill of the art to produce such hybrid neural or paraneural cells without undue experimentation, screen them for the desired traits, such as dopamine secretion, and select those having the best potential for transplantation.

Another source of cells for transplantation according to the present invention is the adrenal medulla. This neural crest-derived tissue has been involved in clinical trials (see Background) to treat PD. Adult monkey adrenal medulla can be cultured in vitro for at least about three weeks as single cells (Notter, M.F. et al., Cell Tiss. Res. 244:69-76 (1986)). These cells respond to NGF by phenotypic alteration from the epithelioid, glandular morphology, to a neuronal morphology in which cells show extensive neuritic arborizations containing microtubular arrays. This neuronal phenotype appears to be critical for long term survival of rat medullary cells in host CNS as well as their integration with host tissue (Stromberg, L. et al., supra). Transplanted adrenal medulla tissue can correct functional deficits resulting from nigrostriatal dopamine depletion in rats (see, for example, Freed et al., 1981, supra). This, however, is thought to be brought about by diffusion of dopamine from the transplant, a phenomenon that decreases three to six months after transplantation. NGF treatment of the transplanted cells induces fiber outgrowth from the transplant into the host and induces a longer lasting behavioral recovery (at least a year). Indeed, without NGF treatment, few chromaffin cells transplanted either to rat (Freed et al., supra) or rhesus monkey caudate nucleus (Morihisia, J.M. et al., Exp. Neurol. 84:643-653 (1984) using prior art techniques survive. Similarly, retinal pigment epithelial cells secrete dopamine and other factors and may be used for brain implants according to the present invention (Li, L. et al., Exp. Eye Res. 47:771-785 (1988); Lui, G.M. et al., Proc. Int'l. Soc. Eye Res. 6:172 (1990); Li, L. et al., Inv. Ophthal. Vis. Sci. 31(Suppl):595 (1990, abstr. 2915-13); Sheedlo, H.J. et al., ibid., abstr. 2916-14; Fektorovich, E.G. et al., ibid. (abstr. 2917-15); Song, M-K et al., supra).

Cells transplanted into the mammalian brain according to the present invention have shown survival in the absence of added growth factors. However, an additional embodiment of the present invention is directed to transplantation of cells attached to a support matrix combined with the treatment, either in vitro prior to transplant, in vivo after transplant, or both, with the appropriate growth/differentiation factor.

Human adrenal medullary cells can be maintained in vitro having the neuronal phenotype for at least nine weeks (Notter, M.F. et al., Schmitt Neurol. Sci. Symp., June 30, 1987, abstr.). NGF induces this conversion from the glandular state. Adrenal medullary cells co-cultured with C6 glioma cells exhibit extensive neuritic arborization and intimate contact with glioma cells. These astrocytic cells, treated with antimitotic agents to inhibit mitosis, are known to produce a growth factor similar to NGF which sustains sympathetic neurons in vitro (Barde, Y.A., Nature 274:818 (1978)). Grafted glial cells may play an important role in functional recovery of neurons and may be an important source of trophic factors (Doering, L.C. et al., J. Neurolog. Sci. 63:183-196 (1984); Gumple, J. et al., Neurosci. Lett. 37: 307-311 (1984)). Therefore, another embodiment of the present invention involves co-culture of neural or paraneural cells with glial cells, their co-incubation with a support matrix, followed by implantation of the support matrix carrying both cell types.

In additional embodiments of the present invention, cells which are not of neural or paraneural origin, but which have been altered to produce a substance of neurological interest, are used. A preferred cell type is a human foreskin fibroblast which is easily obtained and cultured, and survives upon transplantation into the rat brain using the methods of the present invention (see Example III). For use in the present invention, the cells are genetically altered, using methods known in the art, to express neuronal growth factors, neurotransmitters, neuropeptides, or enzymes involved in brain metabolism. (See, for example, Gage, F.H. et al., Neuroscience 23:795-807 (1987); Rosenberg, M.B. et al., Science 242:1575-1578 (1988); Shimohama, S. et al., Mol. Brain Res. 5:271-278 (1989); which are hereby incorporated by reference).

Standard reference works setting forth the general principles of recombinant DNA technology and cell biology, which are hereby incorporated by reference, include Watson, J.D., et al., Molecular Biology of the Gene, Volumes I and II, Benjamin/Cummings Publishing Co., Inc., Menlo Park, CA (1987); Darnell, J.E. et al., Molecular Cell Biology, Scientific American Books, Inc., New York, NY (1986); Lewin, B.M., Genes II, John Wiley & Sons, New York, NY (1985); Old, R.W. et al., Principles of Gene Manipulation: An Introduction to Genetic Engineering, 2nd Ed., University of California Press, Berkeley, CA (1981); Maniatis, T., et al. (Molecular Cloning: A Laboratory Manual, Cold Spring Harbor Press, Cold Spring Harbor, NY (1982)); Sambrook, J. et al. (Molecular Cloning: A Laboratory Manual, 2nd Edition, Cold Spring Harbor Press, Cold Spring Harbor, NY (1989) and Albers, B. et al., Molecular Biology of the Cell, 2nd Ed., Garland Publishing, Inc., New York, NY (1989).

The recombinant DNA molecules useful for the methods of the present invention can be produced through any of a variety of means, such as, for example, DNA or RNA synthesis, or more preferably, by application of recombinant DNA techniques. Techniques for synthesizing such molecules are disclosed by, for example, Wu, R., et al. (Prog. Nucl. Acid. Res. Molec. Biol. 21:101-141 (1978)). Procedures for constructing recombinant molecules are disclosed in detail by Sambrook et al., supra).

Typically, the gene or genes of interest are cloned from a library of expression vectors which has been prepared by cloning DNA or, more preferably, cDNA (from a cell capable of expressing the gene) into an expression vector. The library is then screened for members capable of expressing the gene product of interest, such as a neurotransmitter-synthesizing enzyme, using antibody binding with an antibody specific for the gene product. DNA, or more preferably cDNA, is extracted and purified from a cell which is capable of expressing the gene product of interest. The purified cDNA is fragmentized (by shearing, endonuclease digestion, etc.) to produce a pool of DNA or cDNA fragments. DNA or cDNA fragments from this pool are then cloned into an expression vector in order to produce a library of expression vectors whose members each contain a unique cloned DNA or cDNA fragment.

An "expression vector" is a vector which (due to the presence of appropriate transcriptional and/or translational control sequences) is capable of expressing a DNA (or cDNA) molecule which has been cloned into the vector and of thereby producing a polypeptide or protein. Expression of the cloned sequences occurs when the expression vector is introduced into an appropriate host cell. An appropriate mammalian host cell would be any mammalian cell capable of expressing the cloned sequences. Procedures for preparing cDNA and for producing a genomic library are disclosed by Sambrook et al. (supra).

A DNA sequence encoding a product or products the expression of which is desired for the present invention may be recombined with vector DNA in accordance with conventional techniques, including blunt-ended or staggered-ended termini for ligation, restriction enzyme digestion to provide appropriate termini, filling in of cohesive ends as appropriate, alkaline phosphatase treatment to avoid undesirable joining, and ligation with appropriate ligases. Techniques for such manipulations are disclosed by Sambrook et al., supra. and are well known in the art.

A nucleic acid molecule, such as DNA, is said to be "capable of expressing" a polypeptide if it contains nucleotide sequences which contain transcriptional and translational regulatory information and such sequences are "operably linked" to nucleotide sequences which encode the polypeptide. An operable linkage is a linkage in which the regulatory DNA sequences and the DNA sequence sought to be expressed are connected in such a way as to permit gene expression. The precise nature of the regulatory regions needed for gene expression may vary from organism to organism, but shall in general include a promoter region which, in prokaryotes, contains both the promoter (which directs the initiation of RNA transcription) as well as the DNA sequences which, when transcribed into RNA, will signal the initiation of protein synthesis. Such regions will normally include those 5'-non-coding sequences involved with initiation of transcription and translation, such as the TATA box, capping sequence, CAAT sequence, and the like.

If desired, the non-coding region 3' to the gene sequence coding for the protein may be obtained by the above-described methods. This region may be retained for its transcriptional termination regulatory sequences, such as termination and polyadenylation. Thus, by retaining the 3'-region naturally contiguous to the DNA sequence coding for the protein, the transcriptional termination signals may be provided. Where the transcriptional termination signals are not satisfactorily functional in the expression host cell, then a 3' region functional in the host cell may be substituted.

Two DNA sequences (such as a promoter region sequence and a coding sequence) are said to be operably linked if the nature of the linkage between the two DNA sequences does not (1) result in the introduction of a frame-shift mutation, (2) interfere with the ability of the promoter region sequence to direct the transcription of the coding sequence, or (3) interfere with the ability of the coding sequence to be transcribed by the promoter region sequence. A promoter region would be operably linked to a DNA sequence if the promoter were capable of effecting transcription of that DNA sequence. Thus, to express the protein, transcriptional and translational signals recognized by the host cell are necessary.

A promoter is a double-stranded DNA or RNA molecule which is capable of binding RNA polymerase and promoting the transcription of an "operably linked" nucleic acid sequence. As used herein, a "promoter sequence" is the sequence of the promoter which is found on that strand of the DNA or RNA which is transcribed by the RNA polymerase. A "promoter sequence complement" is a nucleic acid molecule whose sequence is the complement of a "promoter sequence." Hence, upon extension of a primer DNA or RNA adjacent to a single-stranded "promoter sequence complement" or, of a "promoter sequence," a double-stranded molecule is created which will contain a functional promoter, if that extension proceeds towards the "promoter sequence" or the "promoter sequence complement." This functional promoter will direct the transcription of a nucleic acid molecule which is operably linked to that strand of the double-stranded molecule which contains the "promoter sequence" (and not that strand of the molecule which contains the "promoter sequence complement").

The promoter sequences useful for producing cells for the present invention may be either eukaryotic or viral. Suitable promoters are repressible, or, more preferably, constitutive. Useful eukaryotic promoters include the promoter of the mouse metallothionein I gene (Hamer, D., et al., J. Mol. Appl. Gen. 1:273-288 (1982)); the TK promoter of Herpes virus (McKnight, S., Cell 31:355-365 (1982)); the SV40 early promoter (Benoist, C., et al., Nature (London) 290:304-310 (1981)). A preferred promoter, in particular for human fibroblasts, is the collagen promoter (Prockop, D.J. et al., N. End. J. Med. 301:13-23, 77-85 (1979); Eyre, D.R., Science 207:1315-1322 (1980); Martin, G.R. et al., Trends Bioch. Sci. 10:285-287 (1985); which references are hereby incorporated by reference).

Also intended within the scope of the present invention are cells attached to a support matrix, according to the invention, which are frozen and stored in a frozen state using methods well known in the art. Following thawing, the matrix-bound cells are implanted into a recipient brain.

The cells useful in the methods of the present invention may be xenogeneic (= heterologous, i.e., derived from a species different from the recipient), allogeneic (= homologous, i.e., derived from a genetically different member of the same species as the recipient) or autologous, wherein the recipient also serves as the donor.

The number of cells needed to achieve the purposes of the present invention is variable depending on the size, age, weight of the subject, the nature of the underlying disease, other concurrent therapies, and the like. This can be determined by one of skill in the art without undue experimentation. In an adult human, an effective number of cells attached to a support matrix are in the range of about 1 x 10³ to about 1 x 10⁷ cells, more preferably about 5 x 10³ to about 1 x 10⁶ cells. Alternatively, the effective amount of transplanted cells can be determined in terms of mass of cells added to a volume of beads, for example 40 mg of cell mass per ml of beads.

Materials of which the support matrix can be comprised include those materials to which cells adhere following in vitro incubation, and on which cells can grow, and which can be implanted into a mammalian brain without producing a toxic reaction, or an inflammatory or gliosis reaction which would destroy the implanted cells or otherwise interfere with their biological or therapeutic activity. Such materials include, but are not limited to, glass and other silicon oxides, polystyrene, polypropylene, polyethylene, polyvinylidene fluoride, polyurethane, polyalginate, polysulphone, polyvinyl alcohol, acrylonitrile polymers, polyacrylamide, polycarbonate, polypentene, nylon, amylases, gelatin, collagen, natural and modified polysaccharides, including dextrans and celluloses (e.g. nitrocellulose), agar, and magnetite. Either resorbable or non-resorbable materials may be used.

The support matrix of the present invention is distinguished from that described by Aebischer and his colleagues (see above) in that, according to the present invention, the cells are attached to the outer surface of the support; they are not encapsulated within a closed compartment, where their survival would be questionable given the exclusion capacity of the disclosed encapsulating supports. Furthermore, the support matrix of the present invention presents no requirement that the material have particular permeability properties, such as the selective permeability of the Aebischer devices which allow low molecular weight substances to cross but exclude larger molecules (>50 kD).

The configuration of the support is preferably spherical, as in a bead, but may be cylindrical, elliptical, a flat sheet or strip, a needle or pin shape, and the like. A preferred form of support matrix is a glass bead. Another preferred bead is a polystyrene bead. Bead sizes may range from about 10 µm to 1 cm in diameter, preferably from about 90 to about 150 µm. For a description of various microcarrier beads, see, for example, Fisher Biotech Source 87-88, Fisher Scientific Co., 1987, pp. 72-75; Sigma Cell Culture Catalog, Sigma Chemical Co., St. Louis, 1991, pp. 162-163; Ventrex Product Catalog, Ventrex Laboratories, 1989; these references are hereby incorporated by reference. The upper limit on the bead size is dictated by the bead's stimulation of undesired host reactions such as gliosis, which may interfere with the function of the transplanted cells or cause damage to the surrounding brain tissue. Such limitations are readily determinable by one of skill in the art.

To improve cell adhesion, survival and function, the solid matrix may optionally be coated on its external surface with factors known in the art to promote cell adhesion, growth or survival. Such factors include cell adhesion molecules, extracellular matrix, such as, for example, fibronectin, laminin, collagen, elastin, gycosaminoglycans, or proteoglycans (see: Albers, B. supra, pp. 802-834) or growth factors, such as, for example, NGF. Alternatively, if the solid matrix to which the implanted cells are attached is constructed of porous material, the growth- or survival-promoting factor or factors may be incorporated into the matrix material, from which they would be slowly released after implantation in vivo.

In an alternate embodiment of the present invention, cells growing on resorbable matrices, such as, for example, collagen, can be implanted in sites of neurological interest other than the brain, in order to promote neuronal regrowth or recovery. For example, cells attached to the matrix of the invention may be implanted into the spinal cord, or placed in or adjacent to the optic nerve.

The methods of the present invention are useful for treating a number of human neurological disease. Parkinson's Disease can be treated according to the present invention by implanting dopamine-producing cells in the recipient's striatum. Alzheimer's disease (AD) involves a deficit in cholinergic cells in the nucleus basalis. Thus, according to the invention, a subject having AD or at risk therefor may be implanted with cells producing acetylcholine.

Huntington's disease involves a gross wasting of the head of the caudate nucleus and putamen, usually accompanied by moderate disease of the gyrus. A subject suffering from Huntington's disease can be treated by implanting cells producing the neurotransmitters gamma amino butyric acid (GABA), acetylcholine, or a mixture thereof. According to the present invention, the support matrix material to which such cells are attached is preferably implanted into the caudate and putamen.

Epilepsy is not truly a single disease but rather is a symptom produced by an underlying abnormality. One skilled in the art will appreciate that each epileptic subject will have damage or epileptic foci which are unique for the individual. Such foci can be localized using a combination of diagnostic methods well-known in the art, including electroencephalography, computerized axial tomography and magnetic resonance imaging.

A patient suffering from epilepsy can be treated according to the present invention by implanting the support matrix material to which GABA-producing cells are attached into the affected site. Since blockers of glutamate receptors and NMDA receptors in the brain have been used to control experimental epilepsy, cells producing molecules which block excitatory amino acid pathways may be used according to the invention. Thus implantation of cells which have been modified as described herein to produce polyamines, such as spermidine, in larger than normal quantities may be useful for treating epilepsy.

The methods of the present invention are intended for use with any mammal which may experience the beneficial effects of the methods of the invention. Foremost among such mammals are humans, although the invention is not intended to be so limited, and is also applicable to veterinary uses.

Having now generally described the invention, the same will be more readily understood through reference to the following examples which are provided by way of illustration, and are not intended to be limiting of the present invention, 5 unless specified.

### EXAMPLE I

Adrenal chromaffin cells from adult rats were prepared according to known methods (see: Inoue, M. et al., A. J. Physiol. 257 (Cell Physiol. 26):C906-912 (1989)). After sacrifice by nembutal anesthesia followed by decapitation adrenal glands were removed. The medulla was freed of capsular and cortical material by careful microdissection. The tissue was cut into 2 or 3 pieces and incubated for 30 minutes with 0.2% collagenase in calcium-free balanced salt solution containing 140 mM NaCl, 5 mM KCl 5.2 mM MgCl₂, 5 mM HEPES, 10 mM D-glucose, at pH 7.4. During the enzyme treatment, the preparation was shaken gently by bubbling through 99.9% O₂. After digestion, the tissue was washed three or four times with the above salt solution and then triturated gently in a pasteur pipet.

The dissociated chromaffin cells were transferred to cell culture flasks containing serum free medium (Ventrex PC 1) and were cultured overnight at 37°C in a 5% CO₂ atmosphere. Under these conditions, the cells retain their viability for no more than a few days. Sterilized glass beads (Ventrex, 90 micron) were added to the culture flask. The cells attached to the beads and essentially formed a monolayer on the beads. Within 24 hrs. of preparation in vitro, 1 to 5 µl aliquots of the cell-carrying bead suspension were injected into the brains of anesthetized adult rats. Later analysis, at times when the cells would not have survived in culture, the cells were found to be viable in vivo in the site surrounding the injection, (see Figure 1) as determined by immunocytochemical staining for tyrosine hydroxylase according to the Weiner procedure (see: Kilpatrick, D.L. et al., J. Neurochem. 35:679 (1980); Hokfelt, R. et al., Handbook of CHemical Neuroanatomy, Elsevier Science Publishers, Amsterdam (1985)). The glass beads produced no necrotic damage above that typical of the injection itself.

### EXAMPLE II

Adrenal medullae were removed from adult guinea pigs according to methods described in Example I and subjected to the procedure described in Example I. The cells on glass microbeads were injected into rat brain. The cells survived for a period of at least 21 days in a completely healthy state. No signs of immunological rejection were evident.

### EXAMPLE III

Cultured human foreskin fibroblasts were attached to glass microbeads as described above and injected into the rat brain. The cells were localized by immunofluorescence using human IgG which selectively bound to the fibroblasts, followed by a fluorescein-conjugated goat anti-human IgG antibody. The implanted human cells survived for a period of at least 21 days in a completely healthy state. No signs of immunological rejection were evident.

Having now fully described this invention, it will be appreciated by those skilled in the art that the same can be performed within a wide range of equivalent parameters, concentrations, and conditions without departing from the spirit and scope of the invention and without undue experimentation.

While this invention has been described in connection with specific embodiments thereof, it will be understood that it is capable of further modifications. This application is intended to cover any variations, uses, or adaptations of the inventions following, in general, the principles of the invention and including such departures from the present disclosure as come within known or customary practice within the art to which the invention pertains and as may be applied to the essential features hereinbefore set forth as follows in the scope of the appended claims.

The foregoing description of the specific embodiments will so fully reveal the general nature of the invention that others can, by applying current knowledge, readily modify and/or adapt for various applications such specific embodiments without departing from the generic concept, and, therefore, such adaptations and modifications should and are intended to be comprehended within the meaning and range of equivalents of the disclosed embodiments. It is to be understood that the phraseology or terminology employed herein is for the purpose of description and not of limitation.

## Claims

1. Use of a matrix support which has viable cells adhered to the surface of said support for the medicament in preparation of injectable form for use in a method treatment of a neurological disease or dysfunction by implantation or transplantation of said cells into a mammalian brain.

2. Use according to claim 1, wherein the support matrix is made of a material selected from the group consisting of glass, polystyrene, polypropylene, polyethylene, polycarbonate, polypentene, acrylonitrile polymer, nylon, magnetite, natural polysaccharide, a modified polysaccharide and collagen.

3. Use according to claim 1 or 2, wherein said support matrix is a microcarrier bead.

4. Use according to claim 3, wherein said microcarrier bead has a diameter of from about 90 µm to about 150 µm.

5. Use according to any one of claims 1 to 4, wherein said support matrix is glass.

6. Use according to any one of claims 1 to 4, wherein said support matrix is gelatine.

7. Use according to any one of claims 1 to 6, which cells are capable of supplying a neurally active substance.

8. Use according to any one of claims 1 to 7, wherein said cells are selected from the group consisting of retinal pigment epithellial cells, human foreskin fibroblasts, chromaffin cells, cells of neural origin, cells of paraneural origin, cells derived from the adrenal medulla, cells engineered by somatic cell hybridization, and cells that are genetically altered to express neuronal growth factors, neurotransmitters, neuropeptides or enzymes involved in brain metabolism.

9. Use according to claim 8 wherein said cells are retinal pigment epithelial cells.

10. Use according to any one of claims 1 to 9 wherein said mammalian is human.

11. Use according to claim 10, wherein said neurological disease is Parkinson's disease.

12. Use according to claim 11, wherein said cells are dopamine-producing cells.

13. Use according to claim 10, wherein said neurological disease is Alzheimer's disease.

14. Use according to claim 13 wherein said cells produce acetylcholine.

15. Use according to claim 10, wherein said neurological disease is Huntington's disease.

16. Use according to claim 15, wherein said cells produce GABA, acetylcholine, or a mixture thereof.

17. Use according to claim 10, wherein said neurological disease is epilepsy.

18. Use according to claim 17 wherein said cells produce GABA or molecules that block excitatory amino acid pathways.

19. Use according to claim 10, wherein said neurological disease is familial dysautonimia.

20. Use according to claim 10, wherein said neurological disease or dysfunction is caused by traumatic injury to the mammalian brain.

21. A culture of cells on a support matrix, said matrix comprising microbeads having a diameter of from 90 to 150 µM to which the cells adhere, for use in a method of implantation or transplantation of the cells into the mammalian brain.

22. A culture for use according to claim 21, wherein said microbeads are gelatin.

23. A culture for use according to claim 21 or 22, wherein said cells are selected from the group consisting of retinal pigment epithellial cells, human foreskin fibroblasts, chromaffin cells, cells of neural origin, cells of paraneural origin, cells derived from the adrenal medulla, cells engineered by somatic cell hybridization, and cells that are genetically altered to express neuronal growth factors, neurotransmitters, neuropeptides or enzymes involved in brain metabolism.

24. A culture for use according to claim 23 wherein said cells are retinal pigment epithelial cells.

25. A culture for use according to any one of claims 21 to 24 wherein said implantation or transplantation of the cells into the mammalian brain is for the treatment of Parkinson's disease.

26. A culture for use according to any one of claims 21 to 24 wherein cells are dopamine-producing cells.

27. Use of a resorbable matrix support which has viable cells adhered to the surface of said support for the preparation of a medicament in injectable form for use in a method of implantation of cells into a spinal cord for the promotion of neuronal regrowth or recovery.

28. Use according to claim 28 wherein said support comprises collagen.

## Patentansprüche

1. Verwendung eines Matrixträgers, an dessen Oberfläche lebensfähige Zellen haften, zur Herstellung eines Medikaments in injizierbarer Form zur Verwendung in einem Verfahren zur Behandlung einer neurologischen Erkrankung oder Dysfunktion durch Implantation oder Transplantation der Zellen in ein Säugetiergehirn.

2. Verwendung nach Anspruch 1, worin die Trägermatrix aus einem Material besteht, das aus der aus Glas, Polystyrol, Polypropylen, Polyethylen, Polycarbonat, Polypenten, Acrylnitril-Polymer, Nylon, Magnetit, natürlichem Polysaccharid, modifiziertem Polysaccharid und Collagen bestehenden Gruppe ausgewählt ist.

3. Verwendung nach Anspruch 1 oder 2, worin die Trägermatrix ein Mikroträgerkügelchen ist.

4. Verwendung nach Anspruch 3, worin das Mikroträgerkügelchen einen Durchmesser von etwa 90 µm bis etwa 150 µm aufweist.

5. Verwendung nach einem der Ansprüche 1 bis 4, worin die Trägermatrix Glas ist.

6. Verwendung nach einem der Ansprüche 1 bis 4, worin die Trägermatrix Gelatine ist.

7. Verwendung nach einem der Ansprüche 1 bis 6, wobei die Zellen fähig sind, eine neural aktive Substanz bereitzustellen.

8. Verwendung nach einem der Ansprüche 1 bis 7, worin die Zellen aus der aus Netzhautpigment-Epithelzellen, Human-Vorhaut-Fibroblasten, Chromaffinzellen, Zellen neuralen Ursprungs, Zellen paraneuralen Ursprungs, aus dem Nebennierenmark stammenden Zellen, aus durch Somazellen-Hybridisierungsengineering erzeugten Zellen und aus Zellen, die so genetisch verändert sind, dass sie neuronale Wachstumsfaktoren, Neurotransmitter, Neuropeptide oder am Gehirnstoffwechsel beteiligte Enzyme exprimieren, bestehenden Gruppe ausgewählt sind.

9. Verwendung nach Anspruch 8, worin die Zellen Netzhautpigment-Epithelzellen sind.

10. Verwendung nach einem der Ansprüche 1 bis 9, worin das Säugetier ein Mensch ist.

11. Verwendung nach Anspruch 10, worin die neurologische Erkrankung die Parkinson-Krankheit ist.

12. Verwendung nach Anspruch 11, worin die Zellen Dopamin-produzierende Zellen sind.

13. Verwendung nach Anspruch 10, worin die neurologische Erkrankung die Alzheimer-Krankheit ist.

14. Verwendung nach Anspruch 13, worin die Zellen Acetylcholin produzieren.

15. Verwendung nach Anspruch 10, worin die neurologische Erkrankung die Huntington-Krankheit ist.

16. Verwendung nach Anspruch 15, worin die Zellen GABA, Acetylcholin oder ein Gemisch davon produzieren.

17. Verwendung nach Anspruch 10, worin die neurologische Erkrankung Epilepsie ist.

18. Verwendung nach Anspruch 17, worin die Zellen GABA oder Moleküle produzieren, die Erregungs-Aminosäurewege blockieren.

19. Verwendung nach Anspruch 10, worin die neurologische Erkrankung familiäre Dysautonomie ist.

20. Verwendung nach Anspruch 10, worin die neurologische Erkrankung oder Dysfunktion durch traumatische Verletzung am Säugetiergehirn verursacht ist.

21. Zellkultur auf einer Trägermatrix, wobei die Matrix Mikrokügelchen mit einem Durchmesser von 90 bis 150 µm umfasst, an denen die Zellen haften, zur Verwendung in einem Verfahren zur Implantation oder Transplantation der Zellen in das Säugetiergehirn.

22. Kultur zur Verwendung nach Anspruch 21, worin die Mikrokügelchen aus Gelatine bestehen.

23. Kultur zur Verwendung nach Anspruch 21 oder 22, worin die Zellen aus der aus Netzhautpigment-Epithelzellen, Human-Vorhaut-Fibroblasten, Chromaffinzellen, Zellen neuralen Ursprungs, Zellen paraneuralen Ursprungs, aus dem Nebennierenmark stammenden Zellen, aus durch Somazellen-Hybridisierungsengineering erzeugten Zellen und aus Zellen, die so genetisch verändert sind, dass sie neuronale Wachstumsfaktoren, Neurotransmitter, Neuropeptide oder am Gehirnstoffwechsel beteiligte Enzyme exprimieren, bestehenden Gruppe ausgewählt sind.

24. Kultur zur Verwendung nach Anspruch 23, worin die Zellen Netzhautpigment-Epithetzellen sind.

25. Kultur zur Verwendung nach einem der Ansprüche 21 bis 24, worin die Implantation oder Transplantation der Zellen in das Säugetiergehirn zur Behandlung der Parkinson-Krankheit dient.

26. Kultur zur Verwendung nach einem der Ansprüche 21 bis 24, worin die Zellen Dopamin produzierende Zellen sind.

27. Verwendung eines resorbierbaren Matrixträgers, an dessen Oberfläche lebensfähige Zellen haften, zur Herstellung eines Medikaments in injizierbarer Form zur Verwendung in einem Verfahren zur Implantation von Zellen in ein Rückenmark zur Förderung des Nachwachsens oder des Heilungsprozesses von Neuronen.

28. Verwendung nach Anspruch 27, worin der Träger Collagen umfasst.

## Revendications

1. Utilisation d'un support en matrice qui a des cellules viables adhérant à la surface dudit support pour la préparation d'un médicament sous forme injectable pour une utilisation dans un traitement d'une maladie neurologique ou d'un dysfonctionnement par implantation ou transplantation desdites cellules dans un cerveau mammalien.

2. Utilisation selon la revendication 1, où la matrice de support est faite d'un matériau sélectionné dans le groupe consistant en verre, polystyrène, polypropylène, polyéthylène, polycarbonate, polypentène, polymère d'acrylonitrile, nylon, magnétite, polysaccharide naturel, polysaccharide modifié et collagène.

3. Utilisation selon la revendication 1 ou 2, où ladite matrice de support est une micro-perle porteuse.

4. Utilisation selon la revendication 3, où ladite micro-perle porteuse a un diamètre d'environ 90 µm à environ 150 µm.

5. Utilisation selon l'une quelconque des revendications 1 à 4, où ladite matrice de support est du verre.

6. Utilisation selon l'une quelconque des revendications 1 à 4, où ladite matrice de support est de la gélatine.

7. Utilisation selon l'une quelconque des revendications 1 à 6, lesquelles cellules sont capables de fournir une substance neuralement active.

8. Utilisation selon l'une quelconque des revendications 1 à 7, où lesdites cellules sont sélectionnées dans le groupe consistant en cellules épithéliales du pigment rétinien, fibroblastes de prépuce humain, cellules de cromaffine, cellules d'origine neurale, cellules d'origine paraneurale, cellules dérivées de la moelle surrénale, cellules manipulées par une hybridation de cellules somatiques et cellules qui sont génétiquement modifiées pour exprimer des facteurs de croissance des neurones, neurotransmetteurs, neuropeptides ou enzymes impliquées dans le métabolisme du cerveau.

9. Utilisation selon la revendication 8 où lesdites cellules sont des cellules épithéliales du pigment rétinien.

10. Utilisation selon l'une quelconque des revendication 1 à 9, où ledit mammifère est un humain.

11. Utilisation selon la revendication 10, où ladite maladie neurologique est la maladie de Parkinson.

12. Utilisation selon la revendication 11, où lesdites cellules sont des cellules produisant de la dopamine.

13. Utilisation selon la revendication 10, où ladite maladie neurologique est la maladie de Alzheimer.

14. Utilisation selon la revendication 13, où lesdites cellules produisent de l'acétylcholine.

15. Utilisation selon la revendication 10, où ladite maladie neurologique est la maladie de Huntington.

16. Utilisation selon la revendication 15, où lesdites cellules produisent GABA, de l'acétylcholine ou un mélange.

17. Utilisation selon la revendication 10, où ladite maladie neurologique est l'épilepsie.

18. Utilisation selon la revendication 17 où lesdites cellules produisent GABA ou des molécules qui bloquent les trajets d'acides aminés excitateurs.

19. Utilisation selon la revendication 10, où ladite maladie neurologie est une dysautonimie familiale.

20. Utilisation selon la revendication 10, où ladite maladie neurologique ou dysfonctionnement est provoqué par une blessure traumatique du cerveau mammalien.

21. Culture de cellules sur une matrice de support, où ladite matrice comprenant des micro-perles ayant un diamètre de 90 à 150 µM, auxquelles adhèrent les cellules, pour une utilisation dans une méthode d'implantation ou de transplantation des cellules dans le cerveau mammalien.

22. Culture à utiliser selon la revendication 21, où lesdites microperles sont de la gélatine.

23. Culture à utiliser selon la revendication 21 ou 22,où lesdites cellules sont sélectionnées dans le groupe consistant en cellules épithéliales du pigment rétinien, fibroblastes de prépuce humain, cellules de chromaffine, cellules d'origine neurale, cellules d'origine paraneurale, cellules dérivées de la moelle surrénale, cellules manipulées par une hybridation des cellules somatiques et cellules qui sont génétiquement modifiées pour exprimer des facteurs de croissance des neurones, neurotransmetteurs, neuropeptides ou enzymes impliquées dans le métabolisme du cerveau.

24. Culture à utiliser selon la revendication 23 où lesdites cellules sont des cellules épithéliales du pigment rétinien.

25. Culture à utiliser selon l'une quelconque des revendications 21 à 24 où ladite implantation ou transplantation des cellules dans le cerveau mammalien est pour le traitement de la maladie de Parkinson.

26. Culture à utiliser selon l'une quelconque des revendications 21 à 24 où les cellules sont des cellules productrices de dopamine.

27. Utilisation d'un support en matrice résorbable qui a des cellules viables qui adhèrent à la surface dudit support pour la préparation d'un médicament sous forme injectable pour une utilisation dans une méthode d'implantation de cellules dans une moelle épinière pour la promotion de la recroissance neuronale ou la guérison.

28. Utilisation selon la revendication 28 où ledit support comprend du collagène.
